# EUROPEAN PATENT SPECIFICATION

(11) **EP 3 603 633 B1**
(45) Date of publication and mention of the grant of the patent: **16.06.2021**
(21) Application number: 18776637.3
(22) Date of filing: 26.03.2018
(51) Int. Cl.: A61K 31/357, A61K 31/335, A61P 1/00, A61K 9/20, A61K 9/48

(54) **MEDICAL USE OF ARTEMISININ DERIVATIVE FOR TREATING INFLAMMATORY BOWEL DISEASE**
MEDIZINISCHE VERWENDUNG VON ARTEMISININDERIVAT ZUR BEHANDLUNG VON ENTZÜNDLICHEN DARMERKRANKUNGEN
UTILISATION MÉDICALE DE DÉRIVÉ D'ARTÉMISININE POUR TRAITER LA MALADIE INFLAMMATOIRE DES INTESTINS

(30) Priority: 31.03.2017 CN 201710208721
(43) Date of publication of application: 05.02.2020
(73) Proprietor: Shanghai Institute of Materia Medica, Chinese Academy of Sciences, Shanghai 201203 (CN); Jiangsu Zuoyou Medicine Co., Ltd., Suzhou, Jiangsu 215123 (CN)
(72) Inventor: ZUO, Jianping, Shanghai 201203 (CN); TANG, Wei, Shanghai 201203 (CN); YAN, Yuxi, Shanghai 201203 (CN); LI, Ping, Jiangsu 215123 (CN); HE, Shijun, Shanghai 201203 (CN); LIN, Zemin, Shanghai 201203 (CN); YANG, Xiaoqian, Shanghai 201203 (CN); FENG, Chunlan, Shanghai 201203 (CN); ZHU, Fenghua, Shanghai 201203 (CN)
(74) Representative: Grünecker Patent- und Rechtsanwälte PartG mbB
(86) International application number: PCT/CN2018/080416
(87) International publication number: WO 2018/177231

(56) References cited:
- CN-A- 1 814 601
- CN-A- 102 048 728
- JUN-XIA WANG ET AL: "SM905, an artemisinin derivative, inhibited NO and pro-inflammatory cytokine production by suppressing MAPK and NF-[kappa]B pathways in RAW 264.7 macrophages", ACTA PHARMACOLOGICA SINICA, vol. 30, no. 10, 1 October 2009 (2009-10-01), pages 1428-1435, XP055673334, GB ISSN: 1671-4083, DOI: 10.1038/aps.2009.138
- ZE-MIN LIN ET AL: "Artemisinin analogue SM934 attenuate collagen-induced arthritis by suppressing T follicular helper cells and T helper 17 cells", SCIENTIFIC REPORTS, vol. 6, no. 1, 29 November 2016 (2016-11-29), XP055673238, DOI: 10.1038/srep38115

## Description

### TECHNICAL FIELD

The present invention relates to the field of medicine, and in particular, the present invention provides new medical use of four artemisinin derivatives having anti-inflammatory and immunosuppressive activities for treating inflammatory bowel disease. The results of experimental studies in a murine model of ulcerative colitis induced by dextran sulfate sodium (DSS) revealed that intragastric administration of the compounds can effectively alleviate clinical symptoms of ulcerative colitis in model animals such as weight loss, diarrhea, hematochezia etc., reduce pathological damage of colon tissue, inhibit infiltration of inflammatory cells in colon tissue of model mice, and downregulate the levels of pro-inflammatory cytokines. Pharmacodynamic and pharmacologic studies confirmed that this kind of compounds have good immunosuppressive activity and high safety, and can be used as a medicine for the treatment and adjuvant treatment of inflammatory bowel disease and have medical use.

### BACKGROUND ART

Inflammatory bowel disease (IBD) is an idiopathic, chronic intestinal inflammatory disease involving ileum, rectum and colon, including ulcerative colitis (UC) and Crohn's disease (CD), with many complications. Recently, the incidence and prevalence of such diseases are generally on the rise. Chronic colitis and ulcerative colitis are chronic non-specific intestinal inflammatory diseases whose etiology is not clear, and tissue lesions occur in rectum and colon. The diseases have recurrent attacks and the main clinical manifestations are diarrhea, abdominal pain and bloody mucopurulent stool. This type of diseases not only affects the life quality of patients, but is also susceptible to induce colorectal cancer. At present, the drugs for clinical treatment of inflammatory bowel disease are mainly anti-inflammatory and immunosuppressive compounds or biological agents, including aminosalicylic acids, hormones, cyclosporines and tumor necrosis factor monoclonal antibodies and the like. The side effects of these drugs, such as nephrotoxicity and sterol dependence, have led to limitation in their clinical application. Therefore, the development of high-efficient, low-toxic drugs for the treatment of inflammatory bowel disease has a wide range of clinical needs.

Artemisinin is an antimalarial active ingredient extracted from the traditional Chinese medicine *Artemisia annua* L., which is a rare sesquiterpene lactone containing a peroxy group. It is found that it not only has excellent antimalarial effects, but also has good anti-inflammatory and immunosuppressive activity. Since 2000, an immunosuppressive activity-oriented and medicinal chemistry and biology-combined systematic research on artemisinin compounds was carried out. Many series of new artemisinin derivatives were synthesized and a batch of new derivatives which have stronger anti-inflammatory immunosuppressive activities and better compliance in oral administration was found. Based on the above findings, two patent applications were filed and granted, wherein Chinese Patent No. 200680002368.5 discloses an artemisinin derivative, preparation method and use thereof, and a pharmaceutical composition comprising the derivative; and Chinese Patent No. 200680026325.0 discloses a water-soluble artemisinin derivative, and preparation method, pharmaceutical composition and use thereof.

In 2006, we finally determined the representative compound β-aminoarteether maleate (SM934) as a new drug candidate for the autoimmune disease, systemic lupus erythematosus, by studying and evaluating the druggability of a series of artemisinin derivatives in terms of the anti-inflammatory immunosuppressive activity. SM934 is a water-soluble artemisinin derivative with strong immunosuppressive activity and novel mechanism of action. SM934 has high efficacy, low toxicity, good oral absorption and high druggability, and thus has a good application prospect. SM934 was granted 2 Chinese patent rights, 2 US patent rights and 1 Japanese patent right. The research has attracted the attention of international peers. It is believed in Mini-Reviews in Medicinal Chemistry (2007, 7, 411-422) that the developed artemisinin derivative have low toxicity and less adverse effects with a mechanism of action completely different from those of CsA and FK506, and is a promising immunosuppressive drug. It is believed in Chemical Society Reviews (2010, 39(2): 435-454) that the discovery of water-soluble artemisinins is the successful expansion and application of artemisinin compounds in the field of immunology. Mechanism studies revealed that the artemisinin-based compounds mainly act on Th17 cell function and interactively regulated the abnormal activation of signal transduction pathway in immune cells. This work not only develops new immunosuppressive drugs with independent intellectual property rights, but also has important scientific significance and application value to exert the resource advantages of artemisinin in China and to expand the application range of artemisinin compounds.
Ze-Min Lin et al. disclose that artemisinin analogue SM934 attenuates collagen-induced arthritis by suppressing T follicular helper cells and T helper 17 cells in Scientific Reports, vol. 6, no. 1, 29 November 2016.
CN102048728A discloses the application of certain artemisinin derivatives in the preparation of medicaments for treating Crohn disease.

### DISCLOSURE OF INVENTION

It is an object of the present invention to find and provide use of an artemisinin derivative or a pharmaceutically acceptable salt thereof in manufacture of a medicament for the treatment or adjuvant treatment of inflammatory bowel disease.

The subject matter of the invention is as set out in the appended claims.

One aspect of the invention relates to an artemisinin derivative or a pharmaceutically acceptable salt thereof for use in the treatment or adjuvant treatment of an inflammatory bowel disease, wherein the artemisinin derivative is selected from the group consisting of the following compounds:

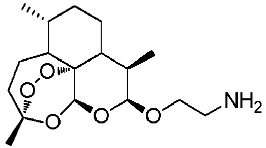

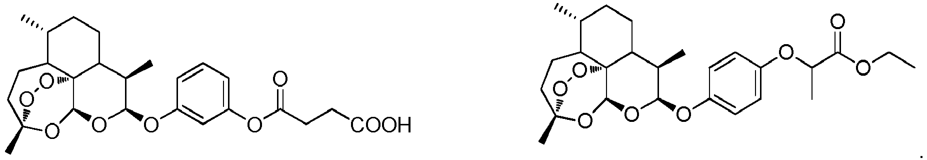

A further aspect of the invention relates to an artemisinin derivative or a pharmaceutically acceptable salt thereof for use in inhibiting infiltration of inflammatory cells into colon tissue and downregulating the level of pro-inflammatory cytokines in colon tissue, wherein the artemisinin derivative is the following compound:

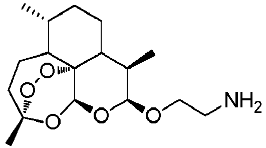

The present specification further discloses the use of an artemisinin derivative or a pharmaceutically acceptable salt thereof in manufacture of a medicament for the treatment or adjuvant treatment of an inflammatory bowel disease, wherein the artemisinin derivative is selected from the group consisting of the following compounds:

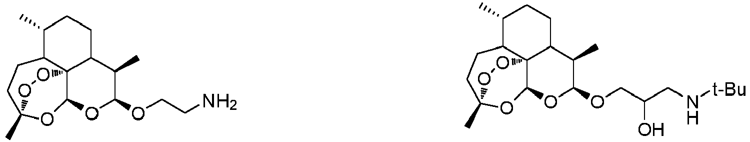

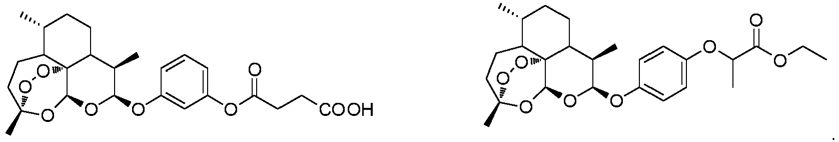

It is proved from the research of the invention that the artemisinin derivatives can effectively inhibit infiltration of inflammatory cells into colon tissue, downregulate the level of pro-inflammatory cytokines in colon tissue, and be used for manufacture of a medicament for the treatment or adjuvant treatment of an inflammatory bowel disease.

The present specification further discloses the use of the artemisinin derivative or a pharmaceutically acceptable salt thereof in manufacture of a medicament for inhibiting infiltration of inflammatory cells into colon tissue and downregulating the level of pro-inflammatory cytokines in colon tissue.

Preferably, in the uses, the pharmaceutically acceptable salt of the artemisinin derivative is the following compound:

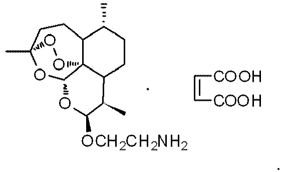

The present specification further discloses that, in the uses, the pharmaceutically acceptable salt of the artemisinin derivative is the following compound:

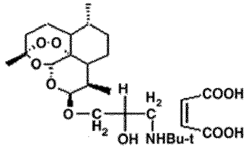

Preferably, in the uses, the medicament may be a tablet, a capsule, a granule, an oral or enema liquid preparation, an intravenous or intramuscular injection or the like.

Further, the present specification discloses a method of treating or adjunctively treating an inflammatory bowel disease, comprising providing a therapeutically effective amount of the artemisinin derivative or a pharmaceutically acceptable salt thereof to a subject in need thereof.

Further, the present specification discloses a method of inhibiting infiltration of inflammatory cells into colon tissue and downregulating the level of pro-inflammatory cytokines in colon tissue, comprising providing a therapeutically effective dosage of the artemisinin derivative or a pharmaceutically acceptable salt thereof to a subject in need thereof.

The present specification also discloses a pharmaceutical composition for treating or adjunctively treating of an inflammatory bowel disease, comprising the artemisinin derivative or a pharmaceutically acceptable salt thereof as an active ingredient.

The present specification also discloses a pharmaceutical composition for inhibiting infiltration of inflammatory cells into colon tissue and downregulating the level of pro-inflammatory cytokines in colon tissue, comprising the artemisinin derivative or a pharmaceutically acceptable salt thereof as an active ingredient.

In the present invention, preferably, the inflammatory bowel disease may include chronic intestinal inflammatory diseases such as ulcerative colitis, Crohn's disease, and chronic enteritis.

### DRAWINGS

Fig. 1 is graphs showing disease activity index scores of mice in various groups during the experiment.
Fig. 2A is a photograph of colon tissues of mice in various groups at the end of the experiment.
Fig. 2B is a graph showing the colon lengths of mice in various groups at the end of the experiment. Compared with the model control group, *, p < 0.05; **, p < 0.01.
Fig. 2C is photographs of pathological sections prepared by fixing the distal colons, which are cut from the distal colon tissues of the mice at the end of the experiment, fixing in formalin, embedding with paraffin, slicing and H&E staining.

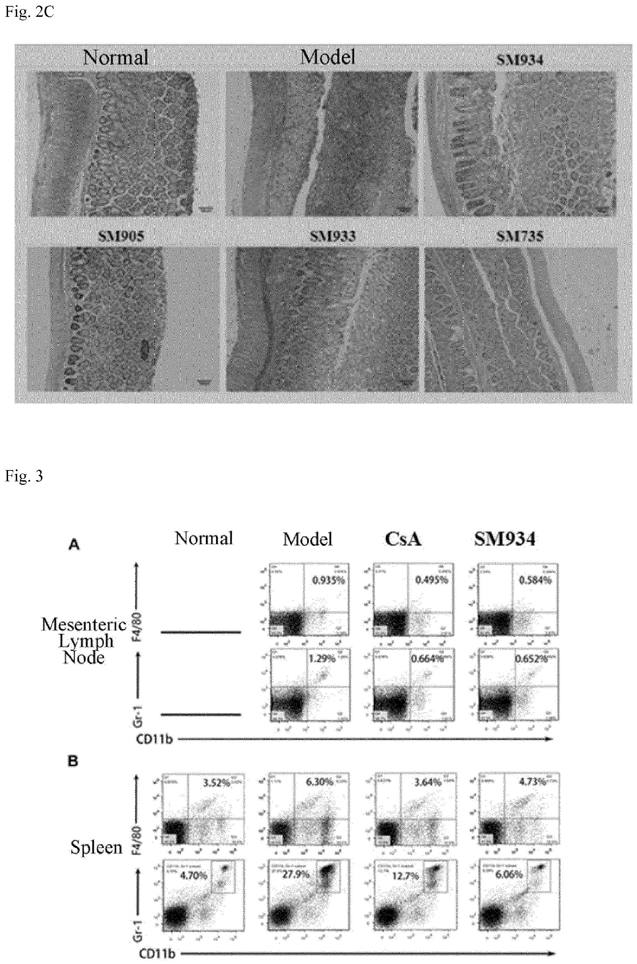

Fig. 3 is graphs showing the results of flow cytometry analysis, in which a single cell suspension was prepared from mesenteric lymph nodes and spleen of mice in various groups taken out at the end of the experiment, and the cells were labeled with the corresponding fluorescent antibody. Fig. 3A is for the isolated mesenteric lymph node cells, and Fig. 3B is for the isolated spleen lymphocytes, they were stained withanti-F4/80 antibody + anti-CD11b antibody (double positive cells were recognized as macrophages), and with anti-Gr-1 antibody + anti-CD 1 1b antibody (double positive cells were identified as neutrophils) respectively.
Fig. 4 is photographs showing the photographing and analyzing results under fluorescence confocal microscopy in which, after the middle segments of colons of mice in various groups were cryosectioned, the nucleus were stained with DAPI fluorescent antibody, and the mononuclear lymphocytes were stained with anti-CD 11b antibody.
Fig. 5 is graphs showing the results of detection of pro-inflammatory cytokine levels in colon tissues of mice in various groups. Compared with the model control group, *, p < 0.05; **,p<0.01; ***, p < 0.001.

### MODE FOR CARRYING OUT THE INVENTION

### EXPERIMENTAL EXAMPLE

Studies on pharmacodynamics and related mechanisms for treating chronic inflammatory bowel diseases were performed on four artemisinin derivatives (SM934, SM905, SM933 and SM735). The compound SM905 is a reference compound.

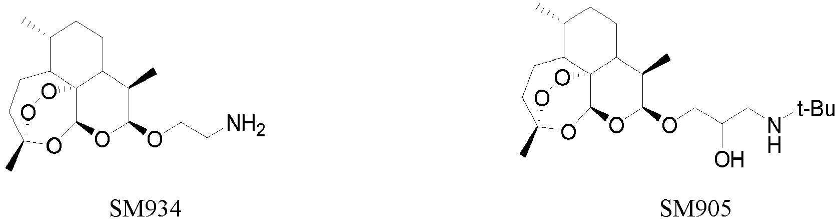

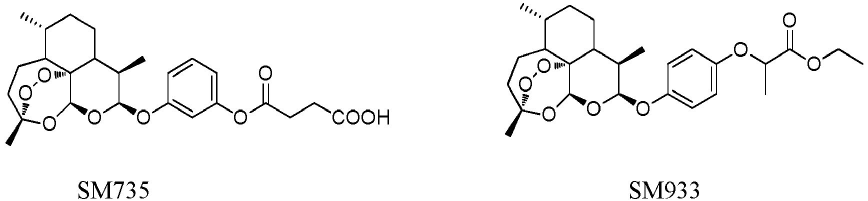

### I. Experimental Materials

Experimental Animals: Inbred female Balb/c mice, about 8 weeks old, Shanghai Slack Laboratory Animals Co., Ltd.

Reagents: dextran sulfate sodium (DSS, MP Biomedicals Biomedical Company, USA); cyclosporine A (CsA, China National Institutes for Food and Drug Control), 5-aminosalicylic acid (5-ASA, Sigma-Aldrich); Flow cytometry antibodies were purchased from BD Pharmingen.

The detailed preparation methods of SM934 and SM905 can be referred to the prior patents of the inventors (CN89109562.4, CN93112454.9) and the published paper J. Med. Chem, 2000, 43: 1635-1640.
SM934:
   Salified with maleic acid. A white crystal. Melting point: 139-141 °C
Elemental Analysis (C₂₁H₃₃NO₉):
   Calculated: C 56.87 H 7.50 N 3.16
   Found: C 56.84 H 7.59 N 3.10
SM905 :
   Salified with maleic acid. A white crystal. Melting point: 171-173 °C
Elemental Analysis (C₂₆H₄₃NO₁₀):
   Calculated: C 58.96 H 8.18 N 2.64
   Found: C 58.92 H 7.91 N 2.59

The detailed preparation methods of SM933 and SM735 can be referred to the contents of the prior patents (CN94113982.4, CN01113407.0) and the patent application CN99807416 of the inventors.
SM73 5:
   Elemental Analysis (C₂₅H₃₂O₉•1/2 H₂O):
   Calculated: C 61.84 H 6.85
   Found: C 62.09 H 6.86
SM933 :
   Elemental Analysis (C₂₆H₃₆O₈):
   Calculated: C 65.53 H 7.61
   Found: C65.46 H 7.66

### II. Experimental Method

### [I] Establishment of DSS-induced ulcerative colitis model in mice and pharmacodynamic evaluation

1. Mice were randomly divided into a normal control group, a model group, a cyclosporin A (CsA) (20 mg/kg) treatment group, a 5-aminosalicylic acid (5-ASA) (50 mg/kg) treatment group, and the artemisinin derivative having anti-inflammatory immunosuppressive activity SM934, SM905, SM933 and SM735 (10 mg/kg each) treatment groups, with 8 mice per group. Except for the mice in the normal control group, for the mice in the other groups including the model group, the cyclosporine A treatment group, the 5-aminosalicylic acid treatment group, and the 4 artemisinin derivative treatment groups, 5% DSS was added to the drinking water for 7 consecutive days for induction of the intestinal inflammation model.
2. From the start of the induction to the end of the experiment, mice in the various treatment groups were given cyclosporine A, 5-aminosalicylic acid 5-ASA or4 artemisinin derivatives (10 mg/kg) respectively by intragastric administration to conduct a therapeutic intervention.
3. Evaluation of disease activity index

During the experiment, the mice in various groups were weighed and examined for fecal occult blood daily, and the disease activity of each mouse was scored according to the following table.

**Disease Activity Index (DAI) score sheet**

| Weight Loss (%) | Stool Consistency | Fecal Occult Blood | Score |
|---|---|---|---|
| 0 | normal | negative | 0 |
| 1∼5 | / | + | 1 |
| 5∼10 | soft | ++ | 2 |
| 10∼20 | / | +++ | 3 |
| >20 | loose | perianal hemorrhage | 4 |

Table 1. Weight loss is divided into 5 levels (0, no decrease or increase; 1, decrease by 1-5%; 2, decrease by 5-10%; 3, decrease by 10-20%; 4, decrease by more than 20%); Fecal hardness is divided into 3 levels (0, normal; 2, soft; 4, loose); fecal occult blood is divided into 5 grades (0, negative; 1, +; 2, ++; 3, +++; 4, perianal hemorrhage).

### [II] Overall and microscopic pathological changes of colon tissue in mice

At the end of the experiment, the colon tissue of the mice was taken to measure its length, and then cut the distal colon, which is fixed in formalin, embedded with paraffin and sectioned, and the sections were stained with H&E to make pathological sections.

### [III] Inflammatory cell detection

1. At the end of the experiment, single cell suspensions were prepared from the spleen and lymph nodes of the mice, and the cells were labeled with the corresponding fluorescent antibodies for flow cytometry analysis.
2. The middle segment of the colon was cryosectioned, stained with the corresponding fluorescent antibody, photographed under a fluorescence confocal microscope and analyzed.

### [IV] Detection of pro-inflammatory cytokine levels in colon tissue

1. The same segment of colon tissue was taken from the mice in various groups for homogenization. After centrifugation, the protein concentration in the homogenate supernatant was quantified with a BCA kit.
2. ELISA was used to detect the content of pro-inflammatory cytokine in the supernatant, which was expressed as the content of cytokine per unit of total protein.

### III. Experimental Findings and Results:

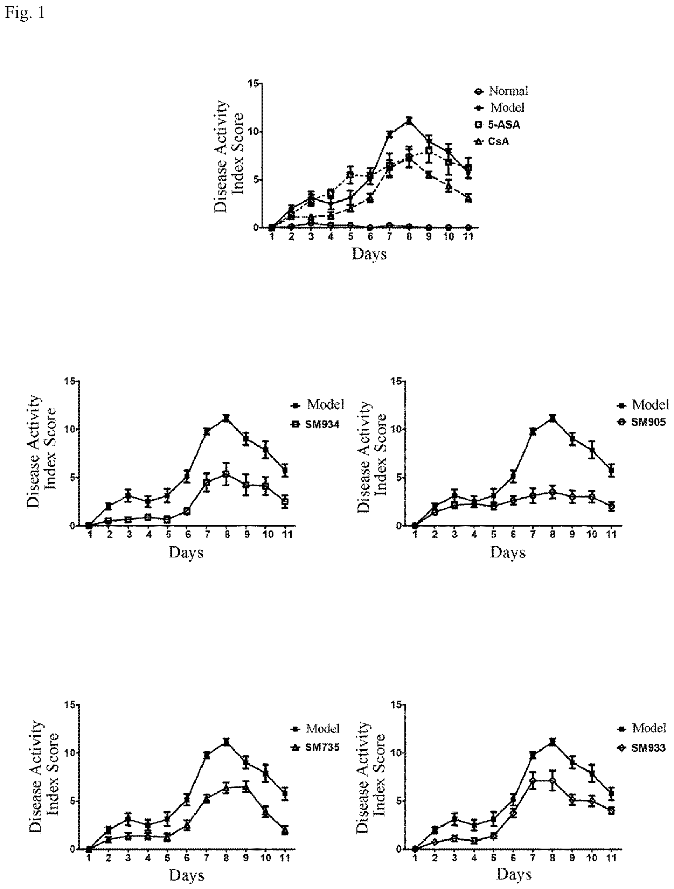

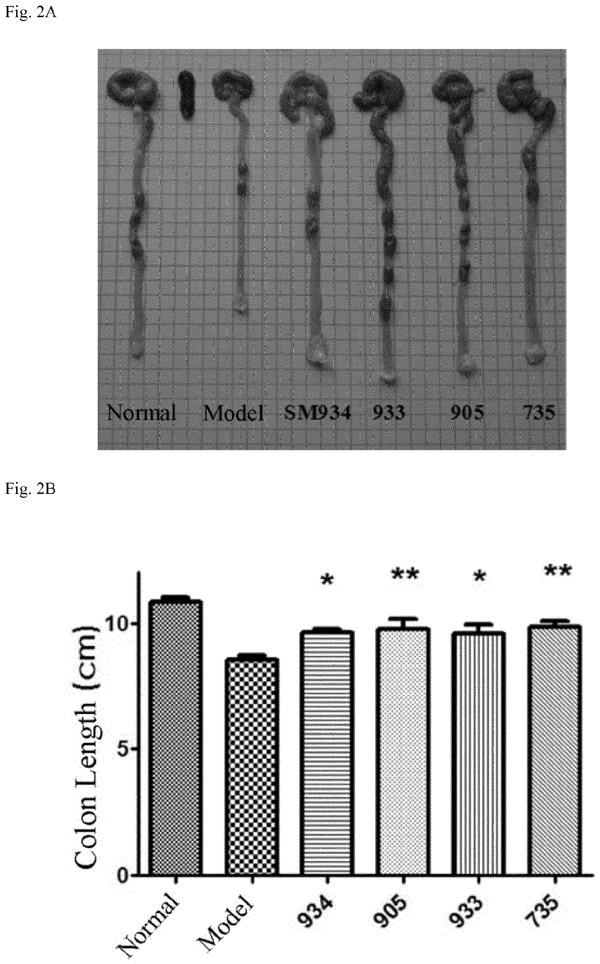

The experimental results in Fig. 1 showed that intragastric administration of the artemisinin derivatives in the present invention can significantly alleviate the signs of DSS-induced inflammatory bowel disease in mice, and the clinic symptoms such as weight loss, diarrhea and hematochezia. The experimental results in Figs. 2A to 2C showed that administration of the artemisinin derivatives of the present invention can significantly restored colon length and attenuate colon pathological damage caused by the DSS-induced inflammation. HE staining of colonic pathological sections revealed that, for the mice in the IBD model group, the colonic glandular structure was disordered, the mucosa and submucosal were infiltrated with the inflammatory cells such as neutrophils, monocytes and multinucleated cells, the mucosa was partially eroded, and some of the crypts were destroyed, while the above changes were significantly alleviated in the mice of the treatment groups.

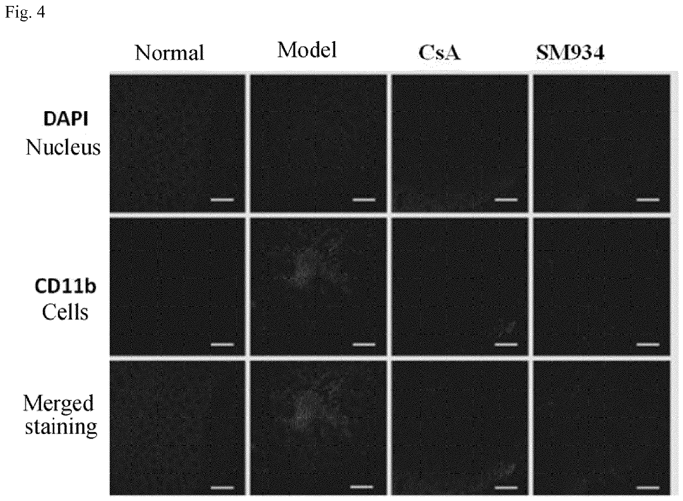

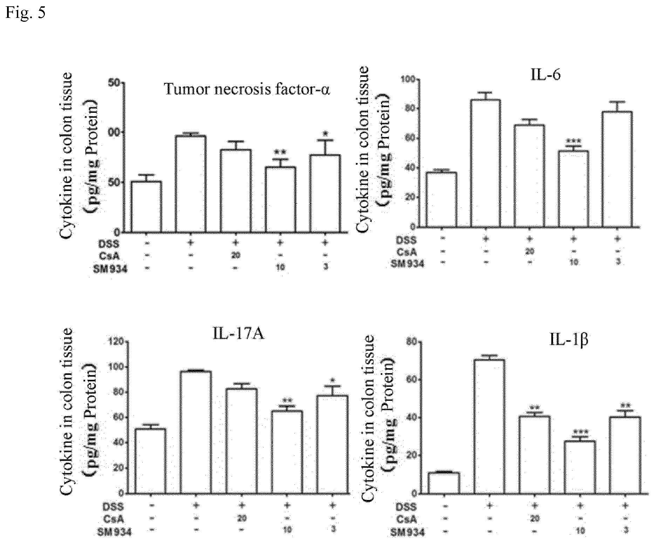

The experimental results in Figs. 3A and 3B showed that administration of SM934 reduced the proportion of macrophages (anti-F4/80+ and CD11b+ double positive cells) and neutrophils (Gr-1+ and CD11b+ double positive cells) in the mesenteric lymph nodes and spleen. The experimental results in Fig. 4 showed that administration of SM934 reduced the infiltration of monocytes (CD11b+ cells) in colon tissue. Pro-inflammatory cytokines (TNF-α, IL-1, IL-6, IL-17, etc.) play a key role in regulating intestinal inflammatory immune responses and participate in the development of the inflammatory bowel disease. The experimental results in Fig. 5 showed that administration of SM934 reduced the levels of pro-inflammatory cytokines in colon tissue.

Therefore, it can be demonstrated from the experimental results of the above-mentioned representative compounds in dextran sulfate sodium(DSS)-induced inflammatory bowel disease model in mice that intragastric administration of these compounds can effectively alleviate clinic symptoms of colitis such as weight loss, diarrhea, hematochezia, etc., reduce pathological damage of colon tissue, inhibit infiltration of inflammatory cells in colon tissue, and downregulate the levels of pro-inflammatory cytokines. It can be confirmed from pharmacodynamic and pharmacologic studies that this type of compounds has good immunosuppressive activity and high safety, and has good application prospect in clinic treatment and adjuvant treatment of inflammatory bowel diseases.

## Claims

1. An artemisinin derivative or a pharmaceutically acceptable salt thereof for use in the treatment or adjuvant treatment of an inflammatory bowel disease, wherein the artemisinin derivative is selected from the group consisting of the following compounds:

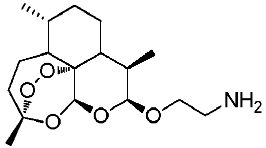

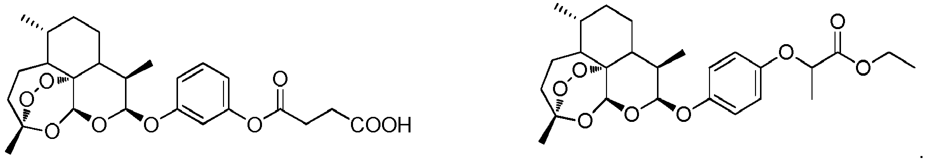

2. An artemisinin derivative or a pharmaceutically acceptable salt thereof for use in inhibiting infiltration of inflammatory cells into colon tissue and downregulating the level of pro-inflammatory cytokines in colon tissue, wherein the artemisinin derivative is the following compound:

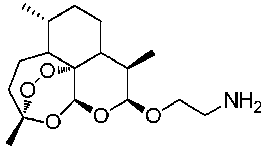

3. The artemisinin derivative or a pharmaceutically acceptable salt thereof for use according to claim 1 or 2, wherein the pharmaceutically acceptable salt of the artemisinin derivative is the following compound:

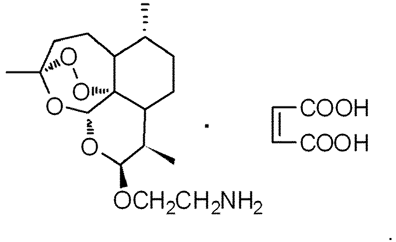

4. The artemisinin derivative or a pharmaceutically acceptable salt thereof for use according to claim 1 or 2, wherein the artemisinin derivative or pharmaceutically acceptable salt thereof is formulated into a medicament, wherein the medicament is a tablet, a capsule, a granule, an oral or enema liquid preparation, or an intravenous or intramuscular injection.

5. The artemisinin derivative or a pharmaceutically acceptable salt thereof for use according to claim 1, wherein the inflammatory bowel disease comprises ulcerative colitis, Crohn's disease, and chronic enteritis.

## Patentansprüche

1. Artemisinin-Derivat oder ein pharmazeutisch verträgliches Salz davon zur Verwendung bei der Behandlung oder adjuvanten Behandlung einer entzündlichen Darmerkrankung, wobei das Artemisinin-Derivat ausgewählt ist aus der Gruppe, bestehend aus den folgenden Verbindungen:

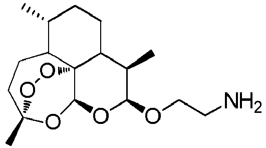

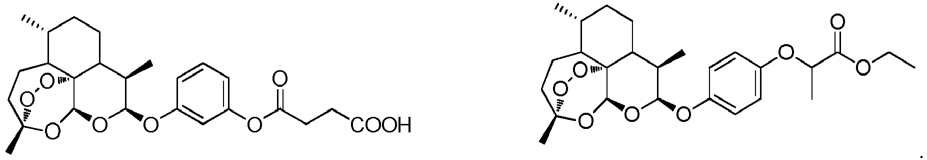

2. Artemisinin-Derivat oder ein pharmazeutisch verträgliches Salz davon zur Verwendung bei der Hemmung der Infiltration von Entzündungszellen in das Dickdarmgewebe und der Herunterregulierung des Spiegels von proinflammatorischen Zytokinen im Dickdarmgewebe, wobei das Artemisinin-Derivat die folgende Verbindung ist:

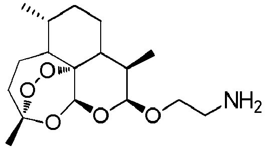

3. Das Artemisinin-Derivat oder ein pharmazeutisch verträgliches Salz davon zur Verwendung nach Anspruch 1 oder 2, wobei das pharmazeutisch verträgliche Salz des Artemisinin-Derivats die folgende Verbindung ist:

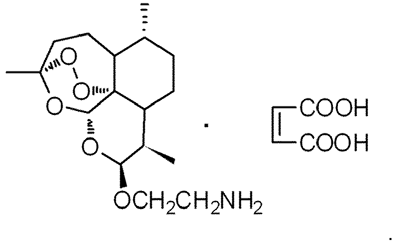

4. Das Artemisinin-Derivat oder ein pharmazeutisch verträgliches Salz davon zur Verwendung nach Anspruch 1 oder 2, wobei das Artemisinin-Derivat oder das pharmazeutisch verträgliche Salz davon zu einem Medikament formuliert ist, wobei das Medikament eine Tablette, eine Kapsel, ein Granulat, ein flüssiges Präparat zum Einnehmen oder als Einlauf, oder eine intravenöse oder intramuskuläre Injektion ist.

5. Das Artemisinin-Derivat oder ein pharmazeutisch verträgliches Salz davon zur Verwendung nach Anspruch 1, wobei die entzündliche Darmerkrankung Colitis ulcerosa, Morbus Crohn und chronische Enteritis umfasst.

## Revendications

1. Dérivé d'artémisinine ou son sel pharmaceutiquement acceptable pour l'utilisation dans le traitement ou le traitement adjuvant d'une maladie de l'intestin inflammatoire, le dérivé d'artémisinine étant sélectionné dans le groupe constitué des composés suivants :

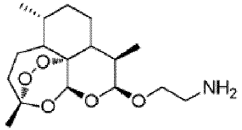

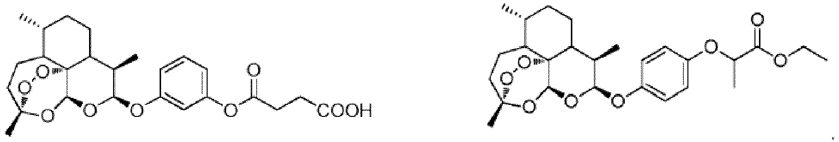

2. Dérivé d'artémisinine ou son sel pharmaceutiquement acceptable pour l'utilisation dans l'inhibition de l'infiltration des cellules inflammatoires dans le tissu du côlon et la régulation à la baisse du niveau de cytokines pro-inflammatoires dans le tissu du côlon, le dérivé d'artémisinine étant le composé suivant :

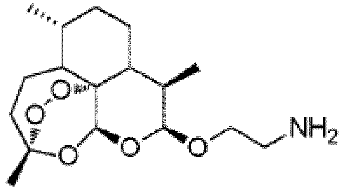

3. Dérivé d'artémisinine ou son sel pharmaceutiquement acceptable pour l'utilisation selon la revendication 1 ou 2, le sel pharmaceutiquement acceptable du dérivé d'artémisinine étant le composé suivant :

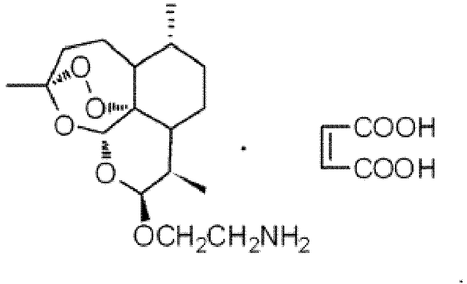

4. Dérivé d'artémisinine ou son sel pharmaceutiquement acceptable pour l'utilisation selon la revendication 1 ou 2, le dérivé d'artémisinine ou son sel pharmaceutiquement acceptable étant formulé en un médicament, le médicament étant un comprimé, une capsule, une granule, une préparation par voie orale ou liquide pour lavement, ou une injection intraveineuse ou intramusculaire.

5. Dérivé d'artémisinine ou son sel pharmaceutiquement acceptable pour l'utilisation selon la revendication 1, la maladie de l'intestin inflammatoire comprenant la rétrocolite hémorragique, la maladie de Crohn, et l'entérite chronique.
